# EUROPEAN PATENT SPECIFICATION

(11) **EP 3 773 023 B1**
(45) Date of publication and mention of the grant of the patent: **30.07.2025**
(21) Application number: 19716492.4
(22) Date of filing: 27.03.2019
(51) Int. Cl.: A61M 15/06, A61M 11/04, A24F 40/53

(54) **ELECTRONIC AEROSOL PROVISION SYSTEM**
ELEKTRONISCHES AEROSOLVERSORGUNGSSYSTEM
SYSTÈME ÉLECTRONIQUE DE FOURNITURE D'AÉROSOLS

(30) Priority: 29.03.2018 GB 201805170
(43) Date of publication of application: 17.02.2021
(60) Divisional application: 25178365.0
(73) Proprietor: Nicoventures Trading Limited, London WC2R 3LA (GB)
(72) Inventor: MOLONEY, Patrick, London WC2R 3LA (GB); KORUS, Anton, Derby DE1 2BU (GB); CHAN, Justin Han Yang, London WC2R 3LA (GB)
(74) Representative: Dehns
(86) International application number: PCT/GB2019/050868
(87) International publication number: WO 2019/186149

(56) References cited:
- EP-A1- 3 216 357
- WO-A1-2016/091658
- WO-A1-2016/187695
- WO-A1-2017/205692
- CA-A1- 2 827 144
- CN-A- 106 666 834
- US-A1- 2014 096 781
- US-A1- 2017 020 191

## Description

### Field

The present disclosure relates to electronic aerosol provision systems such as nicotine delivery systems.

### Background

Electronic aerosol provision systems such as heating products are configured to release one or more compounds by heating, but not burning, a substrate material to generate an aerosol for user inhalation. Generally, the heating products are configured to heat a portion of tobacco or a tobacco derived product (e.g., reconstituted tobacco) to generate the aerosol. The substrate material is usually formed into a rod which is typically surrounded by a paper layer and includes a mouthpiece end, which is an end that the user inhales on (i.e., puts in their mouth) during use. These rods are broadly similar in appearance to combustible cigarettes. The rods are inserted into the aerosol provision device and electrical power is subsequently supplied to the heating element, from a power source such as a battery, to aerosolise portions of the solid substrate in the vicinity of the heating element. Such devices are usually provided with one or more air inlet holes located away from where the user inhales on the system. When a user inhales / sucks on the mouthpiece end of the rods, air is drawn in through the inlet holes, through the rod and past the substrate source. There is a flow path connecting between the aerosol source and an opening in the mouthpiece so that air drawn past the aerosol source continues along the flow path to the mouthpiece opening, carrying some of the aerosol from the aerosol source with it. The aerosol-carrying air exits the aerosol provision system through the mouthpiece for inhalation by the user.

Such rods are formed of low cost components and are generally designed to be thrown away after use (i.e., after the aerosolisable material has been aerosolised). Because the rods are generally reasonably inexpensive to manufacture, any rod of the correct size can be used with the aerosol provision device. However, this has led to counterfeit rods being manufactured in order to be used with the aerosol provision device. These counterfeit rods may not adhere to the strict manufacturing or distributing regulations normally imposed on genuine rods, which can lead to poor quality rods being sold to consumers and used with aerosol provision devices.

Various approaches are described which seek to help address some of these issues.

WO2017205692 discloses an example of a vaporizer and vaporizer systems having a cartridge comprising a cartridge memory.

WO 2016/187695 discloses a vaporizer apparatus for a compressed tablet formed from a plant source material containing medicinal ingredients of therapeutic efficacy. In an embodiment, the apparatus includes: a holder for a compressed tablet; a microprocessor; a controlled air flow; and a controlled heat source; wherein the microprocessor is adapted to control the air flow and the heat source to vaporize the compressed tablet received in the compressed tablet holder at a desired rate. In another embodiment, the vaporizer apparatus includes a carousel for receiving a disc cartridge containing packaged compressed tablets. In still another embodiment, the vaporizer apparatus is adapted to recognize a type of compressed tablet placed into the holder, and to control an air flow and a heat source based on selected therapeutic compounds desired to be released from the recognized type of compressed tablet.

WO2016091658 discloses a method of controlling operation of an inhaling device, the inhaling device comprising a gas flow path through which gas can be drawn by the action of a user puff, a gas flow sensor within the gas flow path and a memory, the method comprising: recording gas flow measurements from the gas flow sensor; comparing the gas flow measurements with the user puff signature stored in memory to provide a correlation score; and enabling or disabling further operation of the device based on a value of the correlation score. This method allows an inhaling device, such as an electrically operated smoking device or a medical inhaler to authenticate a user of the device based on a detected puffing behaviour.

EP3216357 discloses a heating apparatus for heating a cavity inside a chamber. A capsule containing smokeable material may include a readable RFID tag.

CN106666834 discloses a smoking system comprising a smoking set and a smoke cartridge used in cooperation with the smoking set, the smoking set comprises a microcontroller and an execution part and a smoke cartridge reading part which are electrically connected with the microcontroller, the execution part comprises a heating part used for heating the smoke cartridge to generate smoke, and the smoke cartridge comprises a smoke cartridge information carrier carrying the smoke cartridge. The smoke cartridge reading part is configured to obtain smoke cartridge information from the smoke cartridge information carrier, and the microcontroller controls whether the heating part can conduct a heating motion or not according to the smoke cartridge information or whether the heating part can conduct the heating motion or not according to a heating condition corresponding to the smoke cartridge information.

US2017020191 discloses a control body and cartridge that are coupleable with one another to form an aerosol delivery device are provided. The control body comprises a control component and an RFID reader contained within at least one housing. The cartridge comprises at least one heating element and an RFID tag contained within at least one housing. The RFID reader of the control body is coupled to the control component of the control body and configured to communicate with the RFID tag of the cartridge upon coupling of the control body with the cartridge. The control component of the control body is configured to authorize the cartridge for use with the control body based at least in part on communication between the RFID reader and the RFID tag.

### Summary

The invention is defined by the appended claims.

According to a first aspect of certain embodiments there is provided an aerosol provision system for generating aerosol for user inhalation, the system comprising: an aerosol generating article comprising an aerosolisable material, the aerosolisable material being a solid or a gel; and a control unit having a receptacle configured to receive the aerosol generating article, wherein the control unit is configured, in use, to generate aerosol from the aerosolisable material, wherein the aerosol generating article includes a data storage unit configured to store an identifier identifying the aerosol generating article, and wherein the control unit is configured to receive the identifier from the data storage unit and, based on the received identifier, cause the control unit to perform an action.

According to a second aspect of certain embodiments there is provided an aerosol provision device for generating aerosol for user inhalation from an aerosol generating article comprising an aerosolisable material, the aerosolisable material being a solid or a gel and the aerosol generating article including a readable data storage unit configured to store an identifier identifying the aerosol generating article, and wherein the aerosol provision device comprises; a control unit having a receptacle configured to receive the aerosol generating article, wherein the control unit is configured, in use, to generate aerosol from the aerosolisable material, wherein the control unit is configured to perform an action based on an identifier received from the data storage unit of the aerosol generating article.

According to a third aspect of certain embodiments there is provided an aerosol generating article comprising: an aerosolisable material, the aerosolisable material being a solid or a gel; and a readable data storage unit configured to store an identifier identifying the aerosol generating article.

According to a fourth aspect of certain embodiments there is provided a method of identifying an aerosol generating article for use with an aerosol provision device for generating aerosol for user inhalation, the method comprising: receiving, from the readable data storage unit of an aerosol generating article comprising a solid or gel aerosolisable material, an identifier identifying the aerosol generating article; and causing the control unit to perform an action based on the received identifier.

According to a fifth aspect of certain embodiments there is provided an aerosol provision system for generating aerosol for user inhalation, the system comprising: aerosol generating means comprising an aerosolisable material, the aerosolisable material being a solid or a gel; and control means having a receptacle configured to receive the aerosol generating means, wherein the control unit is configured, in use, to generate aerosol from the aerosol generating means, wherein the aerosol generating means includes data storage means configured to store an identifier identifying the aerosol generating means, and wherein the control means is configured to receive the identifier from the data storage means and, based on the received identifier, cause the control means to perform an action.

It will be appreciated that features and aspects of the invention described above in relation to the first and other aspects of the invention are equally applicable to, and may be combined with, embodiments of the invention according to other aspects of the invention as appropriate, and not just in the specific combinations described above.

### Brief Description of the Drawings

Embodiments of the invention will now be described, by way of example only, with reference to the accompanying drawings, in which:
Figure 1 schematically shows an aerosol generating article according to aspects of the present disclosure.
Figure 2 schematically shows an aerosol provision system comprising the aerosol generating article of Figure 1 inserted into an aerosol provision device.
Figure 3 shows an exemplary list of digital identifiers corresponding to the type of aerosolisable material.
Figure 4 schematically shows in more detail an aerosol provision system, wherein the data storage unit is electrically connected to the control circuitry of the aerosol provision device.
Figure 5 schematically shows in more detail an aerosol provision system, wherein the data storage unit is wirelessly coupled to the control circuitry of the aerosol provision device.
Figure 6 shows an exemplary method for generating aerosol, e.g., using the system of Figure 2.

### Detailed Description

Aspects and features of certain examples and embodiments are discussed / described herein. Some aspects and features of certain examples and embodiments may be implemented conventionally and these are not discussed / described in detail in the interests of brevity. It will thus be appreciated that aspects and features of apparatus and methods discussed herein which are not described in detail may be implemented in accordance with any conventional techniques for implementing such aspects and features.

The present disclosure relates to an aerosol provision system, and more specifically to a heating product which is configured to release one or more compounds by heating, but not burning, a substrate material. The substrate material is an aerosolisable material which may be for example tobacco or other non-tobacco products, which may or may not contain nicotine. Substrate materials, which also may be referred to herein as aerosol generating materials, are materials that are capable of generating aerosol for example when heated, irradiated or energized in any other way. A substrate material may be in the form of a solid or gel which may or may not contain nicotine and/or flavourants. In some embodiments the substrate material may comprise a vapour or aerosol generating agent or a humectant, such as glycerol, propylene glycol, triacetin or diethylene glycol. As used herein, the terms "flavour" and "flavourant" refer to materials which, where local regulations permit, may be used to create a desired taste or aroma in a product for adult consumers.

The present disclosure relates to the identification of an aerosol generating article for use with an aerosol provision device. The aerosol generating article comprises a solid or gel aerosolisable material and is generally arranged to provide enough aerosol for the duration of a session, which may be between 8 to 12 inhalations / puffs, although some implementations may allow for up to 20 or 30 puffs depending upon the application at hand. Once consumed, the aerosol generating article is disposed of and replaced with a fresh aerosol generating article. The aerosol generating article comprises a small number of relatively inexpensive components to reduce the cost of the article per available puff.

The aerosol generating article comprises a data storage unit which is configured to store an identifier therein. The identifier can be read by an aerosol provision device when the aerosol generating article is located in / coupled to the aerosol provision device, thus enabling the aerosol provision device to be aware of the aerosol generating article installed. This allows the aerosol provision device the potential to respond to the aerosol generating article to be used with the device, which might include altering the way in which the aerosol generating article is heated, or whether or not the aerosol generating article is permitted to be heated. The use of a data storage unit has numerous advantages. It allows for the aerosol generating article to, visually, look the same as any other but enables identifiers to be associated with the specific aerosol generating article. These identifiers can only be read / interpreted by an appropriate reader (e.g., unlike visual markings which could be read by a reader and a human). Moreover, the use of a data storage unit means that data can be stored securely and, in some cases, may even be encrypted to reduce the chance of counterfeit aerosol generating articles being provided with a genuine identifier.

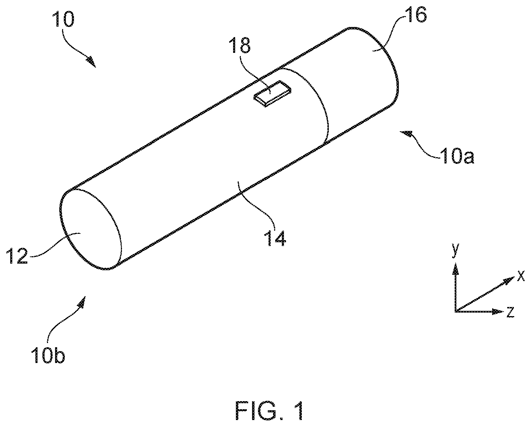

Figure 1 schematically shows, in perspective view, an example of an aerosol generating article 10 according to principles of the present disclosure. The aerosol generating article 10 comprises an aerosolisable material 12, a substrate layer 14, a mouthpiece 16, and a data storage unit 18.

As shown in Figure 1, the aerosol generating article 10 has a generally cylindrical shape. The size of the aerosol generating article 10 is approximately 7 cm in length (along an x-direction) and approximately 0.8 cm in diameter (along a y- / z-direction), although the aerosol generating article 10 may have different dimensions and shapes in different implementations. The aerosol generating article 10 is intended to generate aerosol for user inhalation.

The aerosol generating article 10 includes aerosolisable material 12 which, in the present example, is given as reconstituted tobacco, although it should be appreciated that any of the solid or gel aerosolisable materials discussed above may be used as the aerosolisable material 12 in other implementations. The formation and processing of the aerosolisable material (reconstituted tobacco in the present example) is not the subject of this disclosure and so is not discussed further herein. In the present example, the reconstituted tobacco is formed into a generally rod-shaped / cylindrical element, and around the outer surface of the reconstituted tobacco rod 12 is wrapped a substrate layer 14. The substrate layer 14 in this example is made of paper, but other materials such as card or metal foil (e.g., aluminium foil) may also be used in other implementations. In this example, the substrate layer 14 acts as a physical barrier between the reconstituted tobacco 12 and the external environment, thereby improving the handling of the aerosol generating article 10 by a user. Additionally, the substrate layer 14 may act as an outer wrap to retain the cylindrical rod shape of the reconstituted tobacco 12.

The cylindrical rod has a proximal end 10a and a distal end 10b. In the present example, a mouthpiece 16 is located at the proximal end 10a. The mouthpiece 16 is the part of the aerosol generating article 10 that engages with the lips of a user - in other words, the user places their lips around the mouthpiece 16 during use of the aerosol generating article 10, as explained further below. In some implementations, the substrate layer 14 may be formed of multiple sub-layers stacked one on top of the other (i.e., in the radial direction of article 10), where at least one of the sub-layers extends the entire length of the aerosol generating article 10 and is wrapped around both the aerosolisable material 12 and the mouthpiece 16 to retain the mouthpiece 16 at the proximal end 10a of the aerosol generating article 10. The mouthpiece 16 may be formed of any suitable porous material that is air permeable, e.g., a filter material such as cellulose acetate, a sponge, etc. It should be appreciated however that the mouthpiece 16 is optional and in some implementations the mouthpiece 16 is omitted.

The aerosol generating article 10 further includes a data storage unit 18 which, in this implementation, is positioned on an outer surface of the aerosol generating article 10. More specifically, the data storage unit 18 is located on an outer surface of the substrate layer 14. In the present implementation, the data storage unit 18 is an approximately cuboidal box having various circuitry located therein, which may include a plurality of transistors suitable for storing data. The data storage unit 18 is affixed to the outer surface of the substrate layer 14, e.g., via a suitable adhesive. However, in other implementations, the data storage unit 18 may be located within the aerosol generating article 10 as opposed to on an outer surface of the layer 14. For example, the data storage unit 18 may be located between two sub-layers of the substrate layer 14, or embedded in the aerosol forming material 12 or mouthpiece 16. In some further implementations, the data storage unit 18 may be integrally formed with a component of the aerosol generating article 10, e.g., the layer 14. The data storage unit 18 may be integrally formed during manufacture of the layer 14, for example. The data storage unit 18 is configured to store an identifier which is related to the identity of the aerosol generating article 10. This is explained in more detail below. It should be appreciated that while only one data storage unit 18 is shown in Figure 1, the aerosol generating article 10 may be provided with one or more data storage units 18, each having an identifier (which might be the same identifier for each data storage unit or different identifiers, e.g., two or more different identifiers).

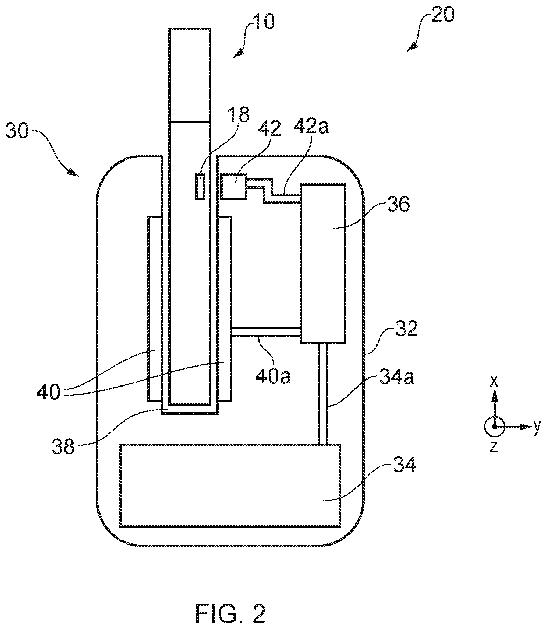

Figure 2 schematically shows, in cross section, an aerosol provision system 20 in accordance with principles of the present disclosure. The aerosol provision system 20 includes the aerosol generating article 10 of Figure 1 in addition to an aerosol provision device 30 (sometimes referred to herein as device part 30). The aerosol provision device 30 includes a housing 32, a power cell 34, a control circuitry 36, a receptacle 38 sized to receive the aerosol generating article 10 of Figure 1, a vaporiser which in this example takes the form of a heater 40 positioned adjacent the receptacle 38 and forming at least a part of the inner surface of the receptacle 38, and a data reader 42.

Figure 2 is described with respect to the reference frame as indicated on the right-hand side of the Figure; however, it should be appreciated that this reference frame is arbitrary and any other reference frame may be used to describe the various orientations and positions of the components of the aerosol provision device 30.

The aerosol provision device 30 includes a housing 32 which defines the outer surface of the device 30. The housing 32 in this example is approximately cuboidal and may have a height in the x-direction of approximately 10 cm, a width in the y-direction of approximately 5 cm, and a thickness in the z-direction of approximately 2 to 3 cm. The corners of the housing are slightly rounded in this example to provide a sleeker appearance and a more ergonomic design. However, it should be appreciated that in other implementations the housing 32 may take a different shape / size.

Inside the housing 32 is provided a power cell 34. The power cell 34 in this example is a rechargeable battery, such as a Lithium Ion battery, which can be recharged when the device 30 is appropriately coupled to an external power source. The power cell 34 is configured to supply electrical power to the control circuitry 36, and ultimately the heater 40, during use of the device 30. The control circuitry 36 is coupled to the power cell 34 via any suitable form of electrical coupling, such as via wires 34a as shown in Figure 2.

The control circuitry 36 is responsible for controlling a number of functions of the device 30. For example, the control circuitry 36 may control the power supply to the heater 40, the charging of the power cell 34 from an external source (e.g., via connection of an external power supply with a USB / microUSB port located in the housing 32, or via an induction based charging mechanism), or any other functionality such as data communication to a host computer (e.g., a personal PC, smartphone, etc.). The control circuitry 36 may include a (micro)controller, processor, ASIC or similar form of control chip in order to realise this control functionality. Moreover, the control circuitry may be formed on or mounted to a printed circuit board (PCB). Note also that the functionality provided by the control circuitry 36 may be split across multiple circuit boards and/or across components which are not mounted to a PCB, and these additional components and/or PCBs can be located as appropriate within the housing. For example, the functionality of the control circuitry for controlling the (re)charging functionality of the battery 32 may be provided separately (e.g. on a different PCB) from the functionality for controlling the discharge (i.e., for providing power to the heater).

The device 30 further includes a receptacle 38 sized to receive at least a part of the aerosol generating article 10. The receptacle in this example is formed as a cylindrical recess extending in the x-direction by a distance approximately two-thirds the length of the aerosol generating article 10, e.g., 5 cm. The aerosol generating article 10 is inserted into the receptacle 38 distal end 10b first. When fully inserted, the distal end of the aerosol generating article 10 rests at the bottom of the receptacle 38 and the proximal end 10a (including the optional mouthpiece 16) protrudes a distance from the surface of the housing 32, e.g., approximately 2 cm of the aerosol generating article 10 is exposed / protrudes from the surface of the housing 32 in this example. In this way, the mouthpiece 16 is presented to the user when the aerosol generating article 10 is inserted into the receptacle 38.

Surrounding the receptacle 38 is provided a heater 40. In this example, the heater 40 is an annular heater 40 (i.e., a hollow cylindrical element) through which the receptacle 38 passes. More specifically, in this example, the inner surface of the annular heater forms a part of the inner surface of the receptacle 38. This arrangement means that the heater can be provided in close proximity to the surface of the aerosol generating article 10, meaning that the heat transfer efficiency from the heater 40 to the aerosol generating article 10 can be improved. The heater 40 in this example is formed from, or at least comprises, an electrically resistive material, e.g., nichrome (NiCr), which generates heat when a current is passed through the resistive material. The supply of power from the power cell 34 to the heater 40 is controlled via the control circuitry 36, as mentioned above. The heater 40 is coupled to the control circuitry 36 via any suitable form of electrical coupling, such as via electrically conductive wires 40a as shown in Figure 2.

In order to generate aerosol for user inhalation, the user must first place the aerosol generating article 10 in the receptacle 38. Thereafter, the aerosol provision system 20 begins supplying power from the power cell 34 to the heater 40 upon activation of the device 30. In the example shown, this is achieved through use of a user actuated button (not shown) provided on the surface of the housing 32. For example, when the button is pressed once, the control circuitry 36 supplies power to the heater 40 for a predetermined time (e.g., the length of a session, such as 2 to 3 minutes). Accordingly, as power is supplied to the heater 40, the temperature of the heater 40 rises. This subsequently heats the aerosol generating article 10 in the receptacle 38 and, more importantly, the aerosolisable material 12 therein to generate a vapour or aerosol. It is important to note that the aerosolisable material 12 is heated and not combusted / burnt. In some implementations, the temperature of the aerosolisable material during heating is between 150 to 300 °C, although it should be appreciated that the precise temperature will depend on the type of aerosolisable material being heated and the construction of the aerosol generating article 10. A user places their lips around the mouthpiece 16 and inhales to draw air from outside the device 30 via an air inlet (not shown) through an opening in the receptacle 38 and through the aerosol generating article 10 (e.g., through the aerosolisable material 12 and generally along a longitudinal axis of the aerosol generating article 10). Air drawn in and along the aerosol generating article 10 collects vaporised particles released from the aerosolisable material 12 as the material 12 is heated to form an aerosol which is then passed along the aerosol generating article 10, through the mouthpiece 16, before entering the user's mouth / lungs.

Generally, the aerosol generating article 10 comprises enough aerosolisable material to last a session, which equates to approximately 8 to 12 user inhalations. The precise quantity of aerosolisable material 12 will be dependent on the type of aerosolisable material 12 in addition to the way in which the device 30 is configured to heat the aerosolisable material. Once the user has finished the session (i.e., the aerosolisable material is spent), the user will remove and dispose of the aerosol generating article 10. To begin a new session, the user inserts a fresh aerosol generating article 10.

As mentioned above, the aerosol generating article 10 according to the present disclosure includes a data storage unit 18, while the device 30 includes a data reader 42. The data storage unit 18 is configured to store an identifier which identifies the aerosol generating article 10. The data reader 42 is configured to read the data storage unit 18 and obtain the identifier therefrom. The data reader 42 is coupled to the control unit via any suitable data connection, e.g., via electrically conductive wires 42a, and is arranged to transmit a signal indicative of the identifier to the control circuitry 36. As will be described in more detail below, the control circuitry 36 receives the signal indicative of the identifier of the aerosol generating article 10 and is arranged to cause the device 30 to perform an action on the basis of the identifier.

The data storage unit 18 in the present example is configured to store a digital representation of the identifier (e.g., a 128-bit identification number). For example, the identifier may be in the form of a binary sequence or of a hexadecimal sequence.

In this example, the data storage unit 18 is programmable, meaning that the identifier can be programmed into the data storage unit 18. That is, the data storage units for two aerosol generating articles 10 may structurally be the same, but can be programmed to store different identifiers accordingly. The programming may be performed before, during, or after manufacture of the aerosol generating article 10. This may simplify the manufacturing process, particularly in the application of the data storage unit 18 to (or in) the aerosol generating article. The data storage unit 18 may be a write once data storage unit 18 (e.g., a write once read many (WORM) data storage unit 18). That is, the data storage unit 18 can be written to once (i.e., when the identifier is applied) and then cannot easily be written to again. In other implementations, the data storage unit 18 may be re-writable (i.e., it can be written to multiple times) depending upon the application at hand.

The identifier is provided to identify the aerosol generating article. This may be on the basis of the type of aerosolisable material 12 of the aerosol generating article 10. Alternatively or additionally, the identifier may identify an origin (geographical and/or manufacturing) of the aerosol generating article 10. Alternatively or additionally, the identifier may uniquely identify the aerosol generating article 10.

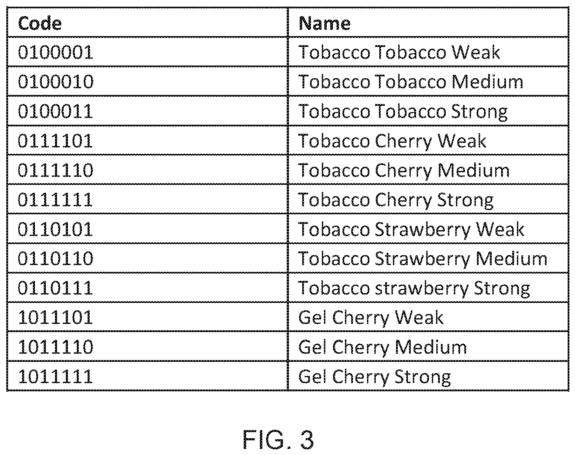

In one implementation, the identifier is related to a substrate material and/or flavour and/or strength of the aerosolisable material. Figure 3 shows an example table including a digital identifier. It should be appreciated that Figure 3 is a non-exhaustive and purely exemplary representation of a possible identifiers. In the examples shown, an aerosol generating article 10 can be associated with a text identifier (i.e., the "Name" column). In this example, each Name is descriptive of the aerosolisable material 12 in the aerosol generating article 10 for ease of description, but it should be appreciated that any other naming convention could be used. In Figure 3, each aerosolisable material 12 is described first by the substrate material to be aerosolised (e.g., Tobacco (such as reconstituted tobacco) or Gel), then by a flavour of the substrate material (e.g., Tobacco flavour, Cherry flavour, Strawberry flavour, etc.), and then by a strength of the active substance, such as nicotine, present in the substrate material (here characterised as Weak, Medium, or Strong, where Medium indicates a higher concentration of active substance than Weak, but less than Strong).

In accordance with this example, each digital identifier (i.e. binary code) is a composite of binary codes associated with each of the categories mentioned above. For example, the material to be aerosolised can be represented as '01' for Tobacco, and '10 for Gel. The flavour can be represented as '000' for Tobacco flavour, '111' for Cherry, and '101' for Strawberry, etc. The strength can be represented as '01' for Weak, '10' for Medium, and '11' for Strong. Accordingly, a seven binary digit code can be created to digitally encode the identifier of the aerosol generating article 10 - e.g., for a tobacco flavoured, reconstituted tobacco aerosol generating article 10 of medium strength, the identifier stored in the data storage unit 18 is '0100010'.

It should be appreciated that the above is purely one way of digitally identifying properties of an aerosol generating article 10. For instance, in some implementations, the device 30 may be configured to only operate with one substrate material, e.g., tobacco, and/or the aerosol generating articles 10 may only be manufactured using one substrate material, in which case the initial two binary digits can be dropped / omitted. In other examples, binary codes may be randomly generated and assigned to the various aerosolisable materials 12 of aerosol generating articles 10.

Regardless of the specific form of the identifier, once the identifier is read by the data reader 42, a signal indicative of the identifier is transmitted to the control circuitry 36. For example, the signal indicative of the identifier may be a modulated signal mirroring the binary code of the identifier. Once received by the control circuitry 36, the control circuitry 36 is configured to interpret the signal and perform an action on this basis of this identifier. In some cases, the control circuitry 36 may be configured to determine whether the identifier belongs to an authenticated article (e.g., by comparing the identifier with one or more stored identifiers in the control circuitry 36, or by comparing the identifier against a remote database of identifiers). In other cases, the control circuitry 36 may additionally or alternatively, interpret the identifier as representing a certain type of aerosol generating article 10, e.g., a tobacco flavoured, reconstituted tobacco aerosol generating article 10 of medium strength. The control circuitry 36 in this example, includes a memory storing a plurality of pre-defined operation modes and the control circuitry 36 is configured to select one of the pre-defined operation modes on the basis of the identifier. This may include, for example, a variety of heating profiles (i.e., temperature vs time profiles). Each of the possible identifiers is linked to a certain heating profile which may be configured to deliver a particular experience to the user when using that aerosol generating article 10. Hence, when the identifier is received, the control circuitry 36 can select the heating profile that is deemed to be suitable for that particular aerosol generating article 10 and then proceeds to heat the aerosol generating article according to that heating profile. It should be appreciated that other operational parameters, besides the heating profile, may also be altered on the basis of the received identifier, e.g., a pressure drop (controlled by altering the size of the air inlet into the device). In other examples, once the identifier has been confirmed as authentic (e.g., if it is present in the memory of the control circuitry 36 it may be deemed authentic), the control circuitry 36 may automatically begin heating of the aerosol generating article 10. In other words, in this implementation, once the article has been identified, the control circuitry is configured to begin heating the article without any further input from the user. This may be the case as soon as the identifier has been confirmed as authentic or after a predetermined delay. Operating in this way may raise the temperature of the aerosol generating article 10 before a user inhales on the article, or until a user input is received, and thus reduce the time required between a user input (e.g., pressing a button or inhaling on the device) and receiving aerosol.

While it has been described that different identifiers are provided for each of the different types of aerosol generating articles 10, it should be appreciated that some identifiers may be used for multiple types of aerosol generating articles 10. This might particularly be the case where, despite the aerosol generating articles 10 comprise different aerosolisable materials 12, the aerosolisable materials are heated according to the same heating profile. In this case, the aerosol generating articles may be grouped into groups with common properties - e.g., suppose the Tobacco Cherry Medium and the Tobacco Strawberry Medium can be heated in the same manner, then these aerosol generating articles can be grouped into a single group and assigned the same identifier. That is, the identifier identifiers that the aerosol generating article 10 belongs to a certain group of aerosol generating articles 10.

In another implementation, instead of being provided with an identifier based on the type of aerosolisable material, the identifier is provided based on the origin of the aerosol generating article 10. For example, each aerosol generating article 10 can be provided with an identifier indicating the origin of the article 10. This could be an identifier indicating that the article is manufacture by a certain manufacturer (such that each manufacturer has a unique identifier), or certain batches of articles 10 could be provided with unique identifiers (such that each batch has a unique identifier). Alternatively or additionally, each aerosol generating article 10 may be provided with a unique identifier (that is, an identifier that is only used on a single article 10).

In these implementations, the device 30 may be configured to operate only when the identifier is considered to be a genuine identifier. For example, assuming all aerosol generating articles 10 manufactured by a certain manufacturer contain an identifier, when the data reader 42 reads the identifier and supplies a signal representative of the identifier to the control circuitry 36, the control circuitry 36 is configured to compare the received identifier with (in this case) a reference identifier obtained in advance. If the two match, the control circuitry 36 is configured to supply power to the heater 40 to heat the aerosol generating article 10. Conversely, if the received identifier does not match the reference identifier, then the control circuitry 36 is configured to not supply power to the heater 40. That is, if the aerosol generating article 10 is found to not include a matching identifier, then the device 30 is configured to not aerosolise the aerosolisable material. The same control mechanism may be present for batches of aerosol generating articles 10, or for individual aerosol generating articles 10, although the number of reference identifiers that the received identifier would have to be checked against is greater for individual articles as opposed to groups of articles 10.

It should be appreciated that while the above has generally described the identifiers relating to type of aerosolisable material and origin as separate, the skilled person will appreciate that these two types of identifiers could be combined in a single identifier. Moreover, a unique identifier may also contain information concerning the type or aerosolisable material and/or the origin of the aerosol generating article.

In the present example, the data storage unit 18 is read by the data reader 42 when the aerosol generating article 10 is inserted in the receptacle 38. The data reader 42 may be controlled by the control circuitry 36 to periodically perform a read operation. If the data storage unit 18 is present or is in range of the data reader 42, the data reader 42 obtains the identifier from the data storage unit 18 and subsequently transmits a signal indicative of the identifier to the control circuitry 36. Alternatively, the data reader 42 may be controlled to read when the user activates the device 30 (e.g., via a push button), which may reduce overall power consumption as the reader 42 is only activated in certain scenarios.

While it has been described above that in certain cases the aerosol provision device 30 may be controlled to not aerosolise the aerosolisable material 12 if the identifier does not match a pre-stored or reference identifier, it should also be appreciated that the device 30 may not aerosolise the aerosolisable material 12 in the even that no identifier can be read by the data reader 42. For instance, should a user insert an aerosol generating article 10 into receptacle 38 that does not include data storage unit 18, then the data reader 42 will not read an identifier and the control circuitry 36 cannot receive the identifier. In this case, the device 30 is configured to prevent the supply of power to the heater 40 even in the event the user depresses the actuation button. Moreover, in some implementations, if no identifier is read within a pre-determined time period, e.g., 1 minute from an initial read operation, then the control unit may be configured to switch off or enter a low power mode to conserve battery power.

In some examples, the device 30 may include an indicator (such as a light or a display) which indicates to the user whether an identifier has been read from aerosol generating article 10 inserted into receptacle 38 or not. In instances where a user inserts a genuine article 10 (i.e., one including a data storage unit 18 with a genuine identifier) but the data reader 42 cannot read the data storage unit 18, the indication of not being able to read the identifier may prompt the user to rotate the aerosol generating article 10 around its longitudinal axis to bring the data storage unit 18 closer to the data reader 42, for example.

Referring back to Figure 2, the data storage unit 18 is provided at a portion of the aerosol generating articles 10 that is not directly heated - specifically, the data storage unit 18 is positioned above the heater 40. The annular heater 40 generally heats a direct region of the aerosol generating article 10 that is encircled by the heater 40 when the aerosol generating article 10 is inserted into the receptacle 38. While heat can travel along the axial direction of the aerosol generating article 10, these regions are not directly heated by the heater 40 itself. Accordingly, the data storage unit 18 is located in these regions that are not directly heated by the heater 40. That is, the data storage unit is provided adjacent to the region of the aerosol generating article 10 to be heated by the heater 40. This may help substantially reduce the influence of the heater 40 on the data storage unit 18 (i.e., reduce the chance of damaging the data storage unit 18 by the heater 40) and may also enable a less heat resilient (and hence more cost efficient) data storage unit 18 to be used.

Generally, the data storage unit 18 described above does not require a power source to store the identifier - that is, the identifier is written into permanent memory. However, in some implementations, the data storage unit 18 may be provided with a power source (this may be integrally formed as part of the data storage unit 18, or provided separately and engaged with the data storage unit 18), which supplies power to non-volatile memory once the identifier is written into the data storage unit 18. This can be advantageous as the power supply can define a lifetime for the aerosol generating article 10 (see below for a more detailed discussion).

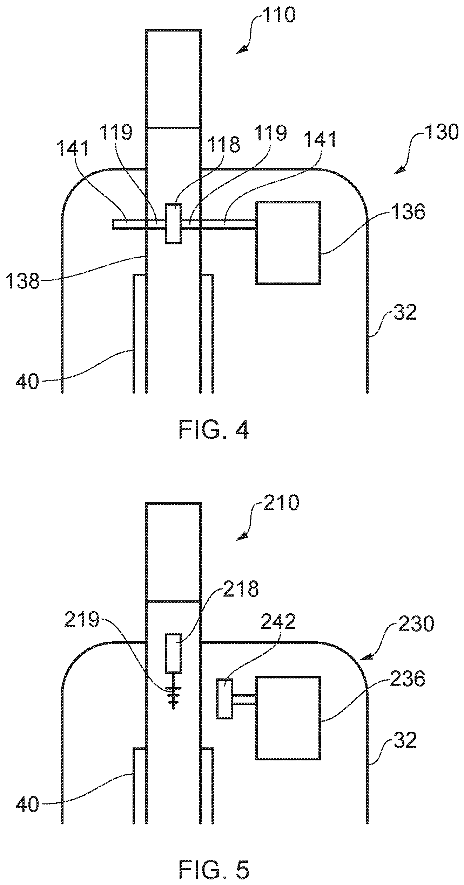

Figures 4 and 5 schematically show more detailed implementations of the data storage unit and data reader, and specifically in terms of the coupling between data storage unit and data reader.

Figure 4 is a schematic representation of an aerosol generating article 110 having a data storage unit 118 that is configured to be electronically read by an aerosol provision device 130.

The aerosol generating article 110 is substantially the same as the aerosol generating article 10 described above, and a discussion of similar features is not provided here. The aerosol generating article 110 includes a data storage unit 118 which is broadly similar to data storage unit 18 described above; however, in Figure 4, the data storage unit 118 is coupled to one or more electrically conductive traces 119. The electrically conductive traces 119 join at one end to the data storage unit 118 and at the other end are exposed. In this example, each the conductive traces 119 are each approximately one-third of the circumference of the aerosol generating article 10, and extend in either direction from the data storage unit 118. Therefore, the traces 119 cover approximately two-thirds of the outer circumference of the aerosol generating article 10. The number of conductive traces 119 used will depend on the type of data storage unit 118 used (e.g., based on the number of inputs and outputs required to read / write to the data storage unit 118).

The device 130 is substantially the same as device 30 described above. However, receptacle 138 in this example includes electrically conductive contacts 141 which are coupled to control circuitry 136. When the aerosol generating article 110 is inserted into the receptacle 138, the exposed ends of the electrical traces 119 are arranged to electrically contact the electrically conductive contacts 141. This permits a signal to be transmitted from the data storage unit 118 to the control circuitry 136 via electrical traces 119 and electrically conductive contacts 141.

In this arrangement, the control circuitry 36 is arranged to perform the function of the data reader 42 described above. Specifically, the control circuitry 136 is configured to read the data storage unit 118 and obtain the identifier stored therein. The precise way in which this is achieved will depend upon the type of data storage unit 118 used and whether or not the data storage unit 118 requires a current to be passed therethrough to be read (in which case the control circuitry 136 will be configured to pass a current through the data storage unit 118 to obtain the identifier) or whether the data storage unit 118 does not require a current to be passed therethrough (in which case the identifier is passed to the control circuitry 136 when the contacts 119 and 141 are coupled).

In this arrangement, the identifier is received via a direct electrical connection between the aerosol generating article 110 and the receptacle 138 of the aerosol provision device 130.

In the implementation shown, the data storage unit 118 and electrical traces 119 are provided on the surface of the aerosol generating article 110. However, in other implementations, the data storage unit 118 and at least a part of the electrical traces 119 may be provided below the outermost surface of the aerosol generating article 110 (e.g., within the aerosolisable material or between sub-layers of the substrate layer. This may help protect the data storage unit 118 and the connection between traces 119 and the data storage unit 118, particularly during handling of the aerosol generating article 110 by a user. However, it should be appreciated that in such implementations, at least part of the electrical traces 119 is exposed (i.e., is provided on the outermost surface of the aerosol generating article 110) in order to achieve electrical contact between the data storage unit 118 and the electrical contacts 141.

In some implementations, the electrical traces 119 and data storage unit 118 are printed directly onto the substrate layer of the aerosol generating article 110. Printing of electronic circuitry may be performed during assembly of the aerosol generating article 110 (i.e., before the substrate layer has been wrapped around the aerosolisable material) or after the aerosol generating article 110 has been formed (i.e., printing onto the curved / wrapped surface of the substrate layer). Although the data storage unit 118 has generally been described as a separate, self-contained unit (i.e., a housing containing circuitry), it should be appreciated that the data storage unit 118 itself may be formed of a number of interconnected electrical components which can be printed directly onto the substrate layer of the aerosol generating article 110.

By printing electrically conductive components directly onto the substrate layer of the aerosol generating article 110, any attempts to transfer the data storage unit 118 to another aerosol generating article (e.g., a counterfeit article) would result in damage to the data storage unit 118 and/or electrical traces 119, resulting in an unsuccessful (or even impossible) transfer of the data storage unit 118 to the counterfeit article. This is particularly useful in preventing counterfeit articles, which may not be manufactured in a highly regulated environment, for being adapted for use with the aerosol provision device 130. In addition, the electronics can be printed in different patterns (and thus store different identifiers) at the time of manufacture.

Figure 5 is a schematic representation of an aerosol generating article 210 having a data storage unit 218 that is configured to be wirelessly read by an aerosol provision device 230.

The aerosol generating article 210 is substantially the same as the aerosol generating article 10 described above, and a discussion of similar features is not provided here. The aerosol generating article 210 includes a data storage unit 218 which operates in a broadly similar manner to data storage unit 18 described above; however, in Figure 5, the data storage unit 218 is electrically coupled to an antenna / transmitter 219. The transmitter 219 is configured to wirelessly transmit a signal indicative of the identifier from data storage unit 218. The transmitter 219 may be formed of any suitable material (e.g., the transmitter may be a metallic strip). The transmitter 219 may be formed on the outer surface of the article 210, e.g., on layer 14. Further, in some instances, the data storage unit 218 may be placed directly on top of the transmitter 219 in order to make an electrical contact between the transmitter 219 and data storage unit 218 (in these cases, the transmitter 219 may have dimensions different to, i.e., greater than, a corresponding dimension of the data storage unit 218). Accordingly, the data storage unit 218 may be provided with suitable electric components to enable the formation of a suitable wireless signal that can be transmitted via transmitter 219; for example, the data storage unit 218 may form part of an integrated circuit (IC) which is coupled to the transmitter 219, where the function of the IC is to generate the wireless signal suitable for transmission via the transmitter 219. The remaining sections of the IC in this example may be generally referred to as a controller / control unit and hence may be configured to control various functions (including signal generation) of the IC.

The device 230 is substantially the same as device 30 described above. However, the device 230 is provided with a wireless receiver 242 connected to the control circuitry 236. The wireless receiver 242 performs the function of the data reader 42 described above in that the receiver 242 is configured to receive the signal indicative of the identifier wirelessly transmitted by the transmitter 219. Once received by the wireless receiver 242, the signal indicative of the identifier is passed to the control circuitry 236 and the control circuitry 236 is configured to alter an aspect of operation of the device 230 on the basis of the identifier (as described above).

The data storage unit 218 and the transmitter 219 are configured to transmit the signal indicative of the identifier in any suitable way using any suitable transmission protocol. In some implementations, the data storage unit 218 and transmitter 219 form an integrated component, for example, an RFID tag configured to transmit a radio frequency, RF, signal (or a modulated RF signal) indicative of the identifier. The data storage unit 218 and the transmitter 219 may be formed on a common substrate (e.g., a semiconductor chip). In these examples, the wireless receiver 242 is a wireless RF receiver, and may be tuned to receive the specific RF frequency. For instance, the RF signal may be generated with signals in the Ultra High Frequency (UHF; approximately 300 to 3,000 MHz), Very High Frequency (VHF; approximately 30 to 300 MHz), High Frequency (HF; approximately 3 to 30 MHz), Medium Frequency (MF; approximately 300 to 3,000 kHz) or Low Frequency (LF; approximately 30 to 300 kHz) range. In some implementations, the RF frequency is in the range of 2.3 to 2.5 GHz, e.g., 2.45 GHz. However, it should be appreciated that other radio based systems, such as Bluetooth^{™}, and/or other radio frequencies different to those given above may also be used in accordance with the principles of the present disclosure.

In some implementations, a power source (not shown) is provided on the aerosol generating article 210. The power source may be provided as a separate component that is individually attached to the aerosol generating article 210 and coupled to the data storage unit 218 / transmitter 219, or the power source may be integrally provided with the data storage unit 218 and/or the transmitter 219 (e.g., the IC may comprise the power source). In this case, the controller may be programmed to transmit the identifier periodically, regardless of whether the aerosol generating article 210 is located in the receptacle 238 of the device 230. (Alternatively, the controller may be configured to transmit the identifier in response to a received signal, as described in more detail below).

This arrangement may increase the cost of goods of the aerosol generating article 210 but may provide a defined lifetime for the aerosol generating article 210 (dependent upon the capacity of the power source and the power consumption of the controller / transmitter 219). Accordingly, once the power source has been sufficiently depleted, either the signal strength becomes too weak to enable reception of the identifier by the receiver 242, or the controller stops functioning and thus stops causing the signal to be transmitted. This means the identifier is not able to be received by the control circuitry 236 and thus the aerosol generating article 210 is unable to be used in the device 230. In other words, the inclusion of a power source may define a period from manufacture in which the article 210 can be used.

In some further implementations, the transmitter 219 and the receiver 242 are both configured to act as transceivers (i.e., they both have transmitting and receiving capabilities). In these implementations, the aerosol generating article 210 is configured to not transmit the identifier (or signal indicative of the identifier) until a request signal transmitted by the device 230 is received by the transceiver 219. In other words, the device 230 is configured to periodically transmit a request signal via the transceiver 242, which signifies a request for the identifier. If no identifier is received within a certain time period, then the device 230 may resend the request signal. The aerosol generating article 210 receives the request signal and then transmits the identifier (or signal indicative of the identifier) via the transceiver 219 upon reception of the request signal. This arrangement ensures that the aerosol generating article only transmits the identifier at a suitable time which additionally can reduce power requirements. The device 230 is configured to not aerosolise the aerosolisable material of the aerosol generating article 210 until such a time as the identifier is received via the transceiver 242.

In other implementations, the aerosol generating article 210 is provided with a wireless power reception module (not shown). The wireless power reception module is configured to receive power wirelessly transmitted by the device 230, e.g., via induction or any other suitable form of wireless power transfer. The wireless power reception module may be integrally provided with the data storage unit 218 and/or the transmitter 219, or the wireless power reception module may be provided as a separate component electronically coupled to the data storage unit 218. That is, the wireless power reception module may form part of the IC. In some instances, the wireless power reception The device 230 is correspondingly provided with a wireless power transmitter (not shown). The wireless power transmitter is accordingly configured to wirelessly transmit power to the wireless power reception module on the aerosol generating article 210. The wireless power transmitter may be configured to transmit power according to any suitable mechanism, e.g., the wireless power transmitter may transmit an RF frequency of 2.45 GHz. Note that the power transmitter and the transmitter 219 may operate at the same or different frequencies. Once power is received, the aforementioned circuitry enables the identifier stored in the data storage unit 218 to be transmitted via the transmitter 219 as previously described. Such an arrangement may be referred to as passive (or passive transmission of the identifier) as the identifier is transmitted only in response to reception of power from a source external to (or separate from) the aerosol generating article 210.

In yet further implementations, the data storage unit 218 and the transmitter 219 may form an integrated circuit having a relatively small size, referred to herein as small-scale IC chips. For example, the areal size of the small-scale IC chip may be less than 6.25 mm², less than 1 mm², or less than 0.1 mm². By way of example only, the small-scale IC chip may have an area extent of 1.0 mm x 1.0 mm or less, 0.75 mm x 0.75 mm or less, or 0.5 mm x 0.5 mm or less. In some implementations, the size of the small-scale IC chip may even be as small as 0.05 mm x 0.05 mm. The thickness of the small-scale IC chip may depend on the construction of, or components included in, the small-scale IC chip but, by way of example, the thickness may be 1.0 mm or less, 0.5 mm or less, or 0.1 mm or less. In some implementations, the thickness may be as thin as 0.005 mm. Generally, small-scale IC chip arrangements may be particularly suited to cases where no power source is provided (either externally to the small-scale IC chip or as part of the small-scale IC chip) which may otherwise generally increase the size of the small-scale IC chip. In other words, such small sizes may generally be achievable in passive small-scale IC chips. Suitable examples of such small-scale IC chips include the RFID DUST developed by Hitachi Ltd of Tokyo, Japan, or the Monza 4 RFID chips manufactured by Impinj Inc. of Washington, USA.

The read range (which is the distance between the transmitter 219 and the receiver 242 above which the receiver is no longer able to receive the identifier) of the IC chip may be dependent upon the size of the transmitter 219 and / or the wireless power reception module. The read range may also be non-uniform with respect to an angular position (that is, the read range may be orientation dependent). The read range of the present implementations may take any value desired; however, because the article 210 and the receiver 242 are generally placed in close proximity of one another, in some implementations the read range may be 30 cm or less, 20 cm or less, or 10 cm or less, or 1 cm or less. Such read ranges are generally possible using IC chips with integrated transmitters (i.e., where the transmitter is or a size comparable to, or less than, the overall size of the IC chip).

Providing a small-scale IC chip enables the small-scale IC chip to be integrated into components forming the aerosol generating article 210. For example, one or more small-scale IC chips may be integrally formed / embedded in the substrate layer 14 (e.g., the paper material forming the substrate layer 14), or in some cases, even in the aerosolisable material 12 of the aerosol generating article 210. As described above, the aerosol generating article 210 may include a substrate layer 14 (such as paper), and the small-scale IC chips can be embedded within the substrate layer 14. Accordingly, during manufacture, the substrate layer 14 can be processed along with the other components forming the aerosol generating article 210 (e.g., the aerosol forming material 12) to form the aerosol generating article 210. In some implementations, the layer 14 is tipping paper including an embedded small-scale IC chip, wherein the layer 14 can be bobbinised (i.e., formed into a bobbin / spool of paper 14) and then used to produce an aerosol generating article 10 according to known techniques / using known machinery. That is, one aspect of the present disclosure is a component for forming an aerosol generating article, wherein the component includes an integrated data storage unit. The small-scale IC chips may be integrated with the layer 14 through a printing method, such as rotogravure, although the skilled person will appreciate that other printing/manufacturing techniques are possible. In some implementations, the small-scale IC chips may be mixed into the pulp used to form the layer 14 prior to formation of the layer 14. In some examples, when the layer 14 is wrapped around, and in some cases adhered to, the aerosol forming material 12 and / or the filter 16, the small-scale IC chips are located at the appropriate position to be read by the receiver 242 when the aerosol generating article 210 is inserted in the device 230. Alternatively, or additionally, one or more small-scale IC chips can be embedded in the aerosolisable material 12 of the article 210, either by embedding the small-scale IC chips within the aerosolisable material 12 during the manufacture of the aerosolisable material (e.g., during the reconstituted tobacco sheet manufacturing process) or by applying the small-scale IC chips during formation of the aerosolisable material 12 (e.g., when forming the sheet into a reconstituted tobacco rod element online during the article 210 making process).

Alternatively, one or more small-scale IC chips can be applied to the surface of the substrate layer 14, e.g., via embedding the small-scale IC chip(s) in a suitable coating material which is subsequently coated on the substrate layer 14, or applied to the aerosolisable material 12 once the aerosolisable material 12 is formed / shaped to the desired shape. The coating may be applied over the whole article 210 or only over a portion (e.g., a portion closer to the proximal end 10a to the distal end 10b, or vice versa, or a middle portion of the article 210). In this regard, the coating may be formed as a slurry, e.g., a slurry comprising the coating material and one or more small-scale IC chips, which is then applied to the substrate layer 14 (however, other techniques for applying the coating may also be used depending on the manufacturing of the article 210). It should also be appreciated that the coating may be applied on either surface of the substrate layer 14 and may be applied before or after assembling the article 210. The coating material may include a liquid adhesive and, in some implementations, the liquid adhesive may be applied to the layer 14 during manufacture of the aerosol generating article 210 (e.g., during wrapping of the layer 14 around the aerosol forming material 12 and / or the filter 16). For instance, the liquid adhesive including small-scale IC chips may adhere ends of the layer 14 to one another. Hence, one aspect of the present disclosure includes an aerosol forming article in which a substrate layer forming the article is adhered using an adhesive including one or more small-scale IC chips.

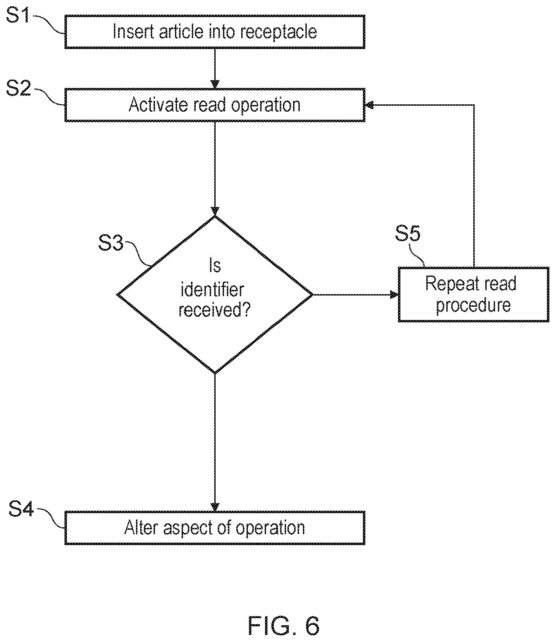

Figure 6 depicts an exemplary method for generating an aerosol for user inhalation from an aerosol generating article 10, 110, 210.

The method begins at step S1, where the user inserts the aerosol generating article 10 into the receptacle 38 of the aerosol provision device 30. This step may be preceded by removal of a previous aerosol generating article, if applicable.

Once the aerosol generating article 10 has been inserted into the receptacle 38 the read operation is activated. As discussed above, this may be triggered by a user activating a button on the exterior housing of the aerosol provision device 30, at which point the data reader 42 begins reading the data storage unit 18, or the data reader 42 may periodically perform a read operation (in which case step S2 is not necessarily only present between steps S1 and S3, but may be periodically present before step S1).

At step S3, the control circuitry determines whether the identifier is received by the control circuitry 36 (i.e., whether the data reader 42 has read the identifier). If yes, then the method proceeds to step S4 where the control circuitry 36 alters an aspect of operation of the device 30. As described above, this could be in terms of beginning a heating operation (in the event the identifier is a genuine identifier) or by altering the way in which the aerosol generating article 10 is heated.

In the alternative, if at step S3 the answer is no, then the method proceeds to repeat the read procedure at step S5 and S2. If the read operation is a periodic read operation, then when transitioning from step S3 to S4, the periodic reading may be temporarily stopped for a set duration, e.g., the duration of a session (e.g., between 5 to 10 minutes). When, for example, the read operation at step S2 is initially performed when the user actuates a button on the housing of the device 30, then if the identifier is initially not received, the method proceeds to activate another instance of the read operation at step S2, until such a time as the identifier is read.

In some cases, the identifier will not be read (as the identifier is not present) and in which case, after a predefined number of read operations (or after a predetermined time period starting from the initial read operation), the device 30 may be configured to indicate that the identifier could not be read (e.g., via an indicator, such as an LED).

Thus, there has been described an aerosol provision system for generating aerosol for user inhalation, wherein the system comprises: an aerosol generating article comprising an aerosolisable material, the aerosolisable material being a solid or a gel; and a control unit having a receptacle configured to receive the aerosol generating article, wherein the control unit is configured, in use, to generate aerosol from the aerosolisable material. The aerosol generating article includes a data storage unit configured to store an identifier identifying the aerosol generating article. The control unit is configured to receive the identifier from the data storage unit and, based on the received identifier, cause the control unit to perform an action.

Although the above has generally described an aerosol generating article 10, 110, 210 in the form of a cylindrical rod, it should be appreciated that the aerosol generating article 10, 110, 210 may take any form as desired. For example, the aerosol generating article may comprise a flat (i.e., not rolled) substrate layer 14 where the aerosolisable material 12 is provided on a surface of the substrate layer 14 (e.g., coated on the layer 14). Other shaped aerosol generating articles may also be possible depending upon the application at hand. It should also be appreciated that the receptacle 38, 138, 238 may be sized to receive the aerosol generating article accordingly. The aerosol generating article 10, 138, 238 may also be provided in the form of a pod, e.g., an aerosolisable material 12 housed in a plastic cage / housing having air holes to allow passage of air therethrough.

Although the above has generally described an aerosol generating article 10, 110, 210 in which the aerosol generating article 10, 110, 210 includes a substrate layer 14. It should be appreciated that the substrate layer 14 of the aerosol generating article 10, 110, 210 may be separate from the aerosol generating material 12 such that the aerosol generating material is removable from the substrate layer 14. In this instance, the aerosolisable material may include a supporting member arranged to hold the aerosolisable material in a manner which enables handling of the aerosolisable material by a user, e.g., the supporting member may be a paper or card tube. The removable substrate layer 14 may function with multiple aerosolisable materials, and includes the data storage unit. That is, the substrate layer 14 includes the data storage unit but can releasably contain or be releasably coupled to multiple portions of aerosolisable material. The substrate layer 14 may be replaced less frequently than the aerosolisable material - that is, the substrate layer 14 may be used for multiple inhalation sessions, where one inhalation session corresponds to generating aerosol from one portion of aerosolisable material. The substrate layer 14, which could be formed from any suitable material such as paper, card, metal, plastics, etc., acts as a sleeve which is inserted into the device and is configured to receive respective portions of aerosolisable material. For such an arrangement, it may be easier and more cost-effective to provide an identifier on or in each sleeve rather than for every portion of aerosolisable material.

It should also be understood that while the above has described a system in which the heater 40 surrounds an outer periphery of the aerosol generating article, the heater may be integrally provided with, or in, the aerosol generating article. For example, the aerosol generating article may comprise a susceptor material (e.g.., mild steel) which is provided in close proximity to the aerosolisable material. Instead of heater 40, the aerosol provision device is instead provided with an inductive work coil which generates a varying magnetic field that can penetrate and thus heat the susceptor material. It should be appreciated that any suitable heating mechanism (or more generally aerosolising mechanism) can be employed with the present disclosure.

It should be appreciated that while the above has described a system in which the data storage unit stores an identifier for identifying the article 10 and causing a control unit to perform an action, the data storage unit 18 may also be configured to store additional data. For example, the data storage unit 18 may be configured to store other information or parameters concerning the article 10, such as a batch number, manufacture number, data of manufacture, etc. In other implementations, the data storage unit 18 may be configured to store additional information such as the heating profile or parameters concerning the heating profile. For example, in this case, when the identifier is transmitted, the heating profile may also be transmitted to the device and, accordingly, the device can heat the consumable according to the transmitted profile. In this case, the identifier may just be used to authenticate the article 10, and not necessarily provide an indication of the flavour / type of the aerosol generating material 12.

While the above described embodiments have in some respects focussed on some specific example aerosol provision systems, it will be appreciated the same principles can be applied for aerosol provision systems using other technologies. That is to say, the specific manner in which various aspects of the aerosol provision system function are not directly relevant to the principles underlying the examples described herein.

In order to address various issues and advance the art, this disclosure shows by way of illustration various embodiments in which the claimed invention(s) may be practiced. The advantages and features of the disclosure are of a representative sample of embodiments only, and are not exhaustive and/or exclusive. They are presented only to assist in understanding and to teach the claimed invention(s). It is to be understood that advantages, embodiments, examples, functions, features, structures, and/or other aspects of the disclosure are not to be considered limitations on the disclosure as defined by the claims and that other embodiments may be utilised and modifications may be made without departing from the scope of the claims. Various embodiments may suitably comprise, consist of, or consist essentially of, various combinations of the disclosed elements, components, features, parts, steps, means, etc. other than those specifically described herein.

## Claims

1. An aerosol provision system for generating aerosol for user inhalation, the system comprising:
an aerosol generating article (10) comprising an aerosolisable material (12), the aerosolisable material being a solid or a gel; and
a control unit (30) having a receptacle (38) configured to receive the aerosol generating article, wherein the control unit is configured, in use, to generate aerosol from the aerosolisable material (12),
wherein:
the aerosol generating article (10) includes a data storage unit (18) configured to store an identifier identifying the aerosol generating article, and the aerosol generating article comprises a substrate (14) and the aerosolisable material (12) is provided adjacent the substrate, wherein the substrate (14) includes at least one of: paper, card, and a metal foil;
**characterised in that**
the control unit (30) is configured to receive the identifier from the data storage unit and, based on the received identifier, cause the control unit to automatically begin heating of the aerosol generating article.

2. The system according to claim 1, wherein the control unit (30) automatically begins heating of the aerosol generating article (10) so as to heat the article before a user inhales on the article.

3. The system according to any of claims 1 or 2, wherein the control unit (30) automatically begins heating of the aerosol generating article (10) so as to heat the article before a user input is received.

4. The system according to any one of claims 1 to 3, wherein the data storage unit (18) is configured to digitally store the identifier, optionally wherein the data storage unit (18) is programmable such that the identifier can be programmed into the data storage unit.

5. The system according to any of claims 1 to 4, wherein the identifier at least one of:
identifies the type of aerosolisable material of the aerosol generating article, identifies the origin of the aerosol generating article, and uniquely identifies the aerosol generating article;
optionally wherein the type corresponds to at least one of a flavour of the aerosolisable material and a concentration of an active substance present in the aerosolisable material.

6. The system according to claim 5, wherein the control unit (30) is configured to operate according to a pre-defined set of operation modes, and wherein the control unit is configured to select one of the operation modes on the basis of the received identifier.

7. The system according to any of claims 1 to 6, wherein the control unit (30) is configured to prevent aerosol generation in the event that no identifier is received or in the event that a received identifier is not recognised as an authorised identifier; and/or.
wherein the control unit (30) further includes an indicator, wherein the indicator is configured to indicate to a user that the control unit has not obtained the identifier.

8. The system according to any of claims 1 to 7, wherein the control unit (30) is configured to heat a portion of the aerosol generating article (10) to produce an aerosol from the aerosolisable material (12), and wherein the data storage unit (18) is located adjacent the portion of the aerosol generating article that is to be heated.

9. The system according to any of claims 1 to 8, wherein the aerosol generating article (10) comprises a plurality of data storage units (18), each data storage unit comprising an identifier.

10. The system according to any of claims 1 to 9, wherein the data storage unit (18) is integrally provided in, or positioned on, the substrate material (14).

11. The system according to any of claims 1 to 10, wherein the aerosol generating article comprises a plurality of electrical contacts (118) coupled to the data storage unit (18), wherein the electrical contacts are exposed on a surface of the aerosol generating article (10), and
wherein the control unit (30) comprises a plurality of electrical contacts (141) configured to electrically couple with the electrical contacts of the aerosol generating article when the aerosol generating article is received by the control unit, wherein the control unit is configured to receive the identifier by applying an electrical current across the electrical contacts of the aerosol forming article;
optionally wherein the electrical contacts and/or data storage unit are printed onto or form a part of a substrate forming an outer surface of the aerosol generating article.

12. The system according to any of claims 1 to 10, wherein the aerosol generating article (10) further comprises a transmitter (219), wherein the transmitter is coupled to the data storage unit and is configured to wirelessly transmit the identifier, and wherein the control unit (30) further comprises a receiver (242) configured to wirelessly receive the identifier transmitted from the transmitter;
optionally wherein the aerosol generating article (10) is configured to transmit the identifier when the aerosol generating article is engaged with the control unit (30);
optionally wherein the transmitter (219) of the aerosol generating article is a transceiver, the transceiver configured to receive a wireless signal from the control unit (30) and, in response to said signal, transmit the identifier.

13. The system according to claim 12, wherein the data storage unit and the transmitter are integrally provided on a base substrate material to form an integrated unit.

14. The system according to claim 13, wherein the integrated units have an areal extent of 6.25 mm² or less, less than 1 mm² or less, or 0.1 mm² or less;
optionally wherein the aerosol generating article includes a plurality of integrated units.

15. The system according to any of claims 1 to 14, wherein the aerosolisable material is selected from one or more of the following group: tobacco, reconstituted tobacco, and gel.

16. A method of identifying an aerosol generating article (10) for use with an aerosol provision device for generating aerosol for user inhalation, the method comprising:
receiving, from a readable data storage unit (18) of an aerosol generating article (10) comprising a solid or gel aerosolisable material (12), an identifier identifying the aerosol generating article, wherein the aerosol generating article (10) comprises a substrate (14) and the aerosolisable material (12) is provided adjacent the substrate, wherein the substrate (14) includes at least one of: paper, card, and a metal foil; and
causing a control unit (30) to automatically begin heating of the aerosol generating article (10)based on the received identifier.

## Patentansprüche

1. Aerosolbereitstellungssystem zum Erzeugen von Aerosol zur Inhalation durch einen Benutzer, wobei das System umfasst
einen Aerosol erzeugenden Artikel (10), der ein aerosolisierbares Material (12) umfasst, wobei das aerosolisierbare Material ein Feststoff oder ein Gel ist; und
eine Steuereinheit (30), die einen Behälter (38) aufweist, der konfiguriert ist, um den Aerosol erzeugenden Artikel aufzunehmen, wobei die Steuereinheit konfiguriert ist, um im Gebrauch Aerosol aus dem aerosolisierbaren Material (12) zu erzeugen, wobei:
der Aerosol erzeugende Artikel (10) eine Datenspeichereinheit (18) beinhaltet, die konfiguriert ist, um eine Kennung zu speichern, die den Aerosol erzeugenden Artikel identifiziert, und der Aerosol erzeugende Artikel ein Substrat (14) umfasst und das aerosolisierbare Material (12) neben dem Substrat bereitgestellt ist, wobei das Substrat (14) mindestens eines beinhaltet von: Papier, Karton und einer Metallfolie;
**dadurch gekennzeichnet, dass**
die Steuereinheit (30) konfiguriert ist, um die Kennung von der Datenspeichereinheit zu empfangen und basierend auf der empfangenen Kennung die Steuereinheit zu veranlassen, automatisch mit dem Erhitzen des Aerosol erzeugenden Artikels zu beginnen.

2. System nach Anspruch 1, wobei die Steuereinheit (30) automatisch mit dem Erhitzen des Aerosol erzeugenden Artikels (10) beginnt, um den Artikel zu erhitzen, bevor ein Benutzer an dem Artikel inhaliert.

3. System nach einem der Ansprüche 1 oder 2, wobei die Steuereinheit (30) automatisch mit dem Erhitzen des Aerosol erzeugenden Artikels (10) beginnt, um den Artikel zu erhitzen, bevor eine Benutzereingabe empfangen wird.

4. System nach einem der Ansprüche 1 bis 3, wobei die Datenspeichereinheit (18) konfiguriert ist, um die Kennung digital zu speichern, wobei die Datenspeichereinheit (18) optional programmierbar ist, sodass die Kennung in die Datenspeichereinheit programmiert werden kann.

5. System nach einem der Ansprüche 1 bis 4, wobei die Kennung mindestens eines von:
den Typ des aerosolisierbaren Materials des Aerosol erzeugenden Artikels identifiziert, den Ursprung des Aerosol erzeugenden Artikels identifiziert, und den Aerosol erzeugenden Artikel eindeutig identifiziert;
wobei der Typ optional mindestens einem von einem Geschmack des aerosolisierbaren Materials und einer Konzentration eines im aerosolisierbaren Material vorhandenen Wirkstoffs entspricht.

6. System nach Anspruch 5, wobei die Steuereinheit (30) konfiguriert ist, um gemäß einem vordefinierten Satz von Betriebsmodi zu arbeiten, und wobei die Steuereinheit konfiguriert ist, um einen der Betriebsmodi auf der Basis der empfangenen Kennung auszuwählen.

7. System nach einem der Ansprüche 1 bis 6, wobei die Steuereinheit (30) konfiguriert ist, um die Aerosolerzeugung zu verhindern, falls keine Kennung empfangen wird oder falls eine empfangene Kennung nicht als autorisierte Kennung erkannt wird; und/oder.
wobei die Steuereinheit (30) weiter einen Indikator beinhaltet, wobei der Indikator konfiguriert ist, um einem Benutzer anzugeben, dass die Steuereinheit die Kennung nicht erhalten hat.

8. System nach einem der Ansprüche 1 bis 7, wobei die Steuereinheit (30) konfiguriert ist, um einen Abschnitt des Aerosol erzeugenden Artikels (10) zu erhitzen, um aus dem aerosolisierbaren Material (12) ein Aerosol herzustellen, und wobei sich die Datenspeichereinheit (18) neben dem Abschnitt des Aerosol erzeugenden Artikels befindet, der zu erhitzen ist.

9. System nach einem der Ansprüche 1 bis 8, wobei der Aerosol erzeugende Artikel (10) eine Vielzahl von Datenspeichereinheiten (18) umfasst, wobei jede Datenspeichereinheit eine Kennung umfasst.

10. System nach einem der Ansprüche 1 bis 9, wobei die Datenspeichereinheit (18) integral im Substratmaterial (14) bereitgestellt, oder darauf positioniert ist.

11. System nach einem der Ansprüche 1 bis 10, wobei der Aerosol erzeugende Artikel eine Vielzahl von elektrischen Kontakten (118) umfasst, die mit der Datenspeichereinheit (18) gekoppelt sind, wobei die elektrischen Kontakte auf einer Oberfläche des Aerosol erzeugenden Artikels (10) freiliegen, und
wobei die Steuereinheit (30) eine Vielzahl von elektrischen Kontakten (141) umfasst, die konfiguriert sind, um elektrisch mit den elektrischen Kontakten des Aerosol erzeugenden Artikels gekoppelt zu werden, wenn der Aerosol erzeugende Artikel von der Steuereinheit empfangen wird, wobei die Steuereinheit konfiguriert ist, um die Kennung durch Anlegen eines elektrischen Stroms über die elektrischen Kontakte des Aerosol bildenden Artikels hinweg zu empfangen;
wobei die elektrischen Kontakte und/oder die Datenspeichereinheit optional auf ein Substrat gedruckt sind oder ein Teil davon bilden, das eine Außenoberfläche des Aerosol erzeugenden Artikels bildet.

12. System nach einem der Ansprüche 1 bis 10, wobei der Aerosol erzeugende Artikel (10) weiter einen Sender (219) umfasst, wobei der Sender mit der Datenspeichereinheit gekoppelt ist und konfiguriert ist, um die Kennung drahtlos zu senden, und wobei die Steuereinheit (30) weiter einen Empfänger (242) umfasst, der konfiguriert ist, um die vom Sender gesendete Kennung drahtlos zu empfangen;
wobei der Aerosol erzeugende Artikel (10) optional konfiguriert ist, um die Kennung zu senden, wenn der Aerosol erzeugende Artikel mit der Steuereinheit (30) in Eingriff steht;
wobei der Sender (219) des Aerosol erzeugenden Artikels optional ein Sendeempfänger ist, der konfiguriert ist, um ein drahtloses Signal von der Steuereinheit (30) zu empfangen und als Reaktion auf das Signal die Kennung zu senden.

13. System nach Anspruch 12, wobei die Datenspeichereinheit und der Sender integral auf einem Basissubstratmaterial bereitgestellt sind, um eine integrierte Einheit zu bilden.

14. System nach Anspruch 13, wobei die integrierten Einheiten eine Flächenausdehnung von 6,25 mm² oder weniger, weniger als 1 mm² oder weniger, oder 0,1 mm² oder weniger aufweisen;
wobei der Aerosol erzeugende Artikel optional eine Vielzahl integrierter Einheiten beinhaltet.

15. System nach einem der Ansprüche 1 bis 14, wobei das aerosolisierbare Material aus einem oder mehreren der folgenden Gruppe ausgewählt ist: Tabak, rekonstituierter Tabak und Gel.

16. Verfahren zum Identifizieren eines Aerosol erzeugenden Artikels (10) zur Verwendung mit einer Aerosolbereitstellungsvorrichtung zum Erzeugen eines Aerosols zur Inhalation durch einen Benutzer, wobei das Verfahren umfasst:
Empfangen, aus einer lesbaren Datenspeichereinheit (18), eines Aerosol erzeugenden Artikels (10), der ein festes oder gelförmiges, aerosolisierbares Material (12) umfasst, wobei eine Kennung den Aerosol erzeugenden Artikel identifiziert, wobei der Aerosol erzeugende Artikel (10) ein Substrat (14) umfasst und das aerosolisierbare Material (12) neben dem Substrat bereitgestellt ist, wobei das Substrat (14) mindestens eines beinhaltet von: Papier, Karton und einer Metallfolie; und
Veranlassen einer Steuereinheit (30), basierend auf der empfangenen Kennung automatisch mit dem Erhitzen des Aerosol erzeugenden Artikels (10) zu beginnen.

## Revendications

1. Système de fourniture d'aérosol pour générer un aérosol pour une inhalation par un utilisateur, le système comprenant :
un article de génération d'aérosol (10) comprenant un matériau aérosolisable (12), le matériau aérosolisable étant un solide ou un gel ; et
une unité de commande (30) présentant un réceptacle (38) configuré pour recevoir l'article de génération d'aérosol, dans lequel l'unité de commande est configurée, lors de l'utilisation, pour générer un aérosol à partir du matériau aérosolisable (12), dans lequel :
l'article de génération d'aérosol (10) inclut une unité de stockage de données (18) configurée pour stocker un identifiant identifiant l'article de génération d'aérosol, et l'article de génération d'aérosol comprend un substrat (14), et le matériau aérosolisable (12) est fourni adjacent au substrat, dans lequel le substrat (14) inclut au moins un de : papier, carton et une feuille métallique ;
**caractérisé en ce que**
l'unité de commande (30) est configurée pour recevoir l'identifiant à partir de l'unité de stockage de données et, sur la base de l'identifiant reçu, amener l'unité de commande à commencer automatiquement à chauffer l'article de génération d'aérosol.

2. Système selon la revendication 1, dans lequel l'unité de commande (30) commence automatiquement à chauffer l'article de génération d'aérosol (10) de manière à chauffer l'article avant qu'un utilisateur n'inhale en se servant de l'article.

3. Système selon l'une quelconque des revendications 1 ou 2, dans lequel l'unité de commande (30) commence automatiquement à chauffer l'article de génération d'aérosol (10) de manière à chauffer l'article avant qu'une entrée utilisateur ne soit reçue.

4. Système selon l'une quelconque des revendications 1 à 3, dans lequel l'unité de stockage de données (18) est configurée pour stocker numériquement l'identifiant, éventuellement dans lequel l'unité de stockage de données (18) est programmable de telle sorte que l'identifiant puisse être programmé dans l'unité de stockage de données.

5. Système selon l'une quelconque des revendications 1 à 4, dans lequel l'identifiant réalise au moins une de :
l'identification du type de matériau aérosolisable de l'article de génération d'aérosol, l'identification de l'origine de l'article de génération d'aérosol et l'identification de manière unique de l'article de génération d'aérosol ;
éventuellement dans lequel le type correspond à au moins un d'un arôme du matériau aérosolisable et d'une concentration d'une substance active présente dans le matériau aérosolisable.

6. Système selon la revendication 5, dans lequel l'unité de commande (30) est configurée pour fonctionner selon un ensemble prédéfini de modes de fonctionnement, et dans lequel l'unité de commande est configurée pour sélectionner l'un des modes de fonctionnement sur la base de l'identifiant reçu.

7. Système selon l'une quelconque des revendications 1 à 6, dans lequel l'unité de commande (30) est configurée pour empêcher la génération d'aérosol dans le cas où aucun identifiant n'est reçu ou dans le cas où un identifiant reçu n'est pas reconnu comme un identifiant autorisé ; et/ou
dans lequel l'unité de commande (30) inclut en outre un indicateur, dans lequel l'indicateur est configuré pour indiquer à un utilisateur que l'unité de commande n'a pas obtenu l'identifiant.

8. Système selon l'une quelconque des revendications 1 à 7, dans lequel l'unité de commande (30) est configurée pour chauffer une partie de l'article de génération d'aérosol (10) pour produire un aérosol à partir du matériau aérosolisable (12), et dans lequel l'unité de stockage de données (18) est située adjacente à la partie de l'article de génération d'aérosol qui doit être chauffée.

9. Système selon l'une quelconque des revendications 1 à 8, dans lequel l'article de génération d'aérosol (10) comprend une pluralité d'unités de stockage de données (18), chaque unité de stockage de données comprenant un identifiant.

10. Système selon l'une quelconque des revendications 1 à 9, dans lequel l'unité de stockage de données (18) est intégralement fournie dans, ou positionnée sur, le matériau de substrat (14).

11. Système selon l'une quelconque des revendications 1 à 10, dans lequel l'article de génération d'aérosol comprend une pluralité de contacts électriques (118) couplés à l'unité de stockage de données (18), dans lequel les contacts électriques sont exposés sur une surface de l'article de génération d'aérosol (10), et
dans lequel l'unité de commande (30) comprend une pluralité de contacts électriques (141) configurés pour être couplés électriquement aux contacts électriques de l'article de génération d'aérosol lorsque l'article de génération d'aérosol est reçu par l'unité de commande, dans lequel l'unité de commande est configurée pour recevoir l'identifiant en appliquant un courant électrique à travers les contacts électriques de l'article de formation d'aérosol ;
éventuellement dans lequel les contacts électriques et/ou l'unité de stockage de données sont imprimés sur ou font partie d'un substrat formant une surface extérieure de l'article de génération d'aérosol.

12. Système selon l'une quelconque des revendications 1 à 10, dans lequel l'article de génération d'aérosol (10) comprend en outre un émetteur (219), dans lequel l'émetteur est couplé à l'unité de stockage de données et est configuré pour émettre sans fil l'identifiant, et dans lequel l'unité de commande (30) comprend en outre un récepteur (242) configuré pour recevoir sans fil l'identifiant émis par l'émetteur ;
éventuellement dans lequel l'article de génération d'aérosol (10) est configuré pour émettre l'identifiant lorsque l'article de génération d'aérosol est en prise avec l'unité de commande (30) ;
éventuellement dans lequel l'émetteur (219) de l'article de génération d'aérosol est un émetteur-récepteur, l'émetteur-récepteur étant configuré pour recevoir un signal sans fil à partir de l'unité de commande (30) et, en réponse audit signal, émettre l'identifiant.

13. Système selon la revendication 12, dans lequel l'unité de stockage de données et l'émetteur sont fournis intégralement sur un matériau de substrat de base pour former une unité intégrée.

14. Système selon la revendication 13, dans lequel les unités intégrées présentent une étendue aréale de 6,25 mm² ou moins, de moins de 1 mm² ou moins, ou de 0,1 mm² ou moins ;
éventuellement dans lequel l'article de génération d'aérosol inclut une pluralité d'unités intégrées.

15. Système selon l'une quelconque des revendications 1 à 14, dans lequel le matériau aérosolisable est choisi parmi un ou plusieurs du groupe suivant : tabac, tabac reconstitué et gel.

16. Procédé d'identification d'un article de génération d'aérosol (10) pour une utilisation avec un dispositif de fourniture d'aérosol pour générer un aérosol pour une inhalation par un utilisateur, le procédé comprenant :
la réception, à partir d'une unité de stockage de données lisibles (18) d'un article de génération d'aérosol (10) comprenant un matériau aérosolisable solide ou en gel (12), d'un identifiant identifiant l'article de génération d'aérosol, dans lequel l'article de génération d'aérosol (10) comprend un substrat (14) et le matériau aérosolisable (12) est fourni adjacent au substrat, dans lequel le substrat (14) inclut au moins un de : papier, carton et une feuille métallique ; et
le fait d'amener une unité de commande (30) à commencer automatiquement à chauffer l'article de génération d'aérosol (10) sur la base de l'identifiant reçu.
